# EUROPÄISCHE PATENTSCHRIFT

(11) **EP 0 590 551 B1**
(45) Veröffentlichungstag und Bekanntmachung des Hinweises auf die Patenterteilung: **30.10.1996**
(21) Anmeldenummer: 93115512.1
(22) Anmeldetag: 25.09.1993
(51) Int. Cl.: A61K 31/50, A61K 31/55

(54) **Neue therapeutische Verwendungen von Phthalazinon-Derivaten**
New therapeutic uses of phthalazinone-derivatives
Nouvelles utilisations thérapeutiques des dérivés de phthalazinone

(30) Priorität: 02.10.1992 DE 4233113
(43) Veröffentlichungstag der Anmeldung: 06.04.1994
(73) Patentinhaber: ASTA Medica Aktiengesellschaft, D-01277 Dresden (DE)
(72) Erfinder: Engel, Jürgen, Prof., D-63755 Alzenau (DE); Kutscher, Bernhard, Dr., D-63477 Maintal (DE); Fleischhauer, Ilona, Dr., D-63075 Offenbach (DE); Szelenyi, Stefan, Prof., D-90571 Schwaig (DE); Metzenauer, Peter, Dr., D-63584 GrÜndau (DE); Werner, Ulrich, Dr., D-56357 Miehlen (DE)

(56) Entgegenhaltungen:
- EP-A- 0 222 191
- DE-A- 4 000 070
- J MOL CELL CARDIOL Bd. 22, Nr. SIII , 1990 Seite S81 L. VIRAG 'EFFECT OF ANTIHISTAMINES ON THE ELECTRICAL ACTIVITY OF RABBIT PURKINJE FIBRES'
- EUR J PHARMACOLOG Bd. 164 , 1989 Seiten 547 - 553 P.A. MOLYDAS ET AL. 'Azelastin effects on electrical and mechanical activities of guinea pig papillary muscles'
- J CHROMATOGRAPHY Bd. 603 , 1992 Seiten 105 - 109 N. MANO ET AL. 'Conalbumin-conjugated silica gel, a new chiral stationary phase for high-performance liquid chromatography'
- ARZNEIM.-FORSCH./DRUG RES. Bd. 40, Nr. 12 , 1990 Seiten 1295 - 1299 K. TASAKA ET AL. 'Central effect of the potent long-acting H1-antihistamine Levocabastine'
- J. CLIN. BIOCHEM. NUTR. Bd. 6 , 1989 Seiten 205 - 211 S. YAMAMOTO ET AL. 'Circumvention of adriamycin-resistance of leukemia cells by the anti-allergic drug azelastin'
- J. CLIN. BIOCHEM. NUTR. Bd. 14 , 1993 Seiten 7 - 15 Y. FUKUDA ET AL. 'Circumvention of multidrug resistance of leukemia cells by the anti-allergic agent azelastine'
- CHEMICAL ABSTRACTS, vol. 111, no. 9, 28. August 1989, Columbus, Ohio, US; abstract no. 70968, 'Azelastine as therapeutic and prophylactic agent for ischemic heart disease', Seite 82; & JP-A-63 218 622

## Beschreibung

Phthalazinon-Derivate sind in der Literatur bekannt. So wird zum Beispiel Azelastin (INN) erfolgreich in die Therapie und Prophylaxe des Asthma bronchiale und der allergischen und saisonalen Rhinitis eingesetzt.

Als Nachfolgeverbindung wurde Flezelastin in Form des racemischen Gemisches entwickelt. Die Herstellung findet sich im EP 222 191 beschrieben.

Es wurde nun überraschend gefunden, daß die Phthalazinon-Derivate gemäß der allgemeinen Formel I 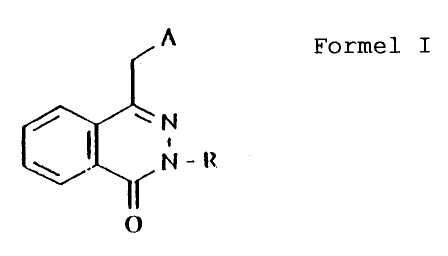
- wobei A =: Phenyl (unsubstituiert, ein- oder mehrfach halogensubstituiert), H oder C₁-C₃-Alkyl, 2-Furyl oder 2-Thienyl bedeutet und
- R =: 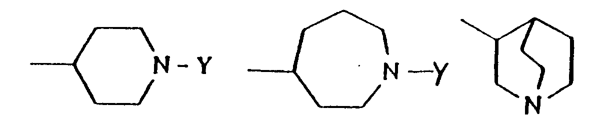
- mit Y =: C₁-C₆ - Alkyl, wobei der Alkylrest mit einem (substituierten) Phenylring substituiert sein kann, wenn R ein Azepinring bedeutet, dann ist Y = C₁-C₆ Alkyl, substituiert mit einem (substituierten) Phenylring
sowie deren physiologisch unbedenklichen Säureadditionssalze und die Verbindungen 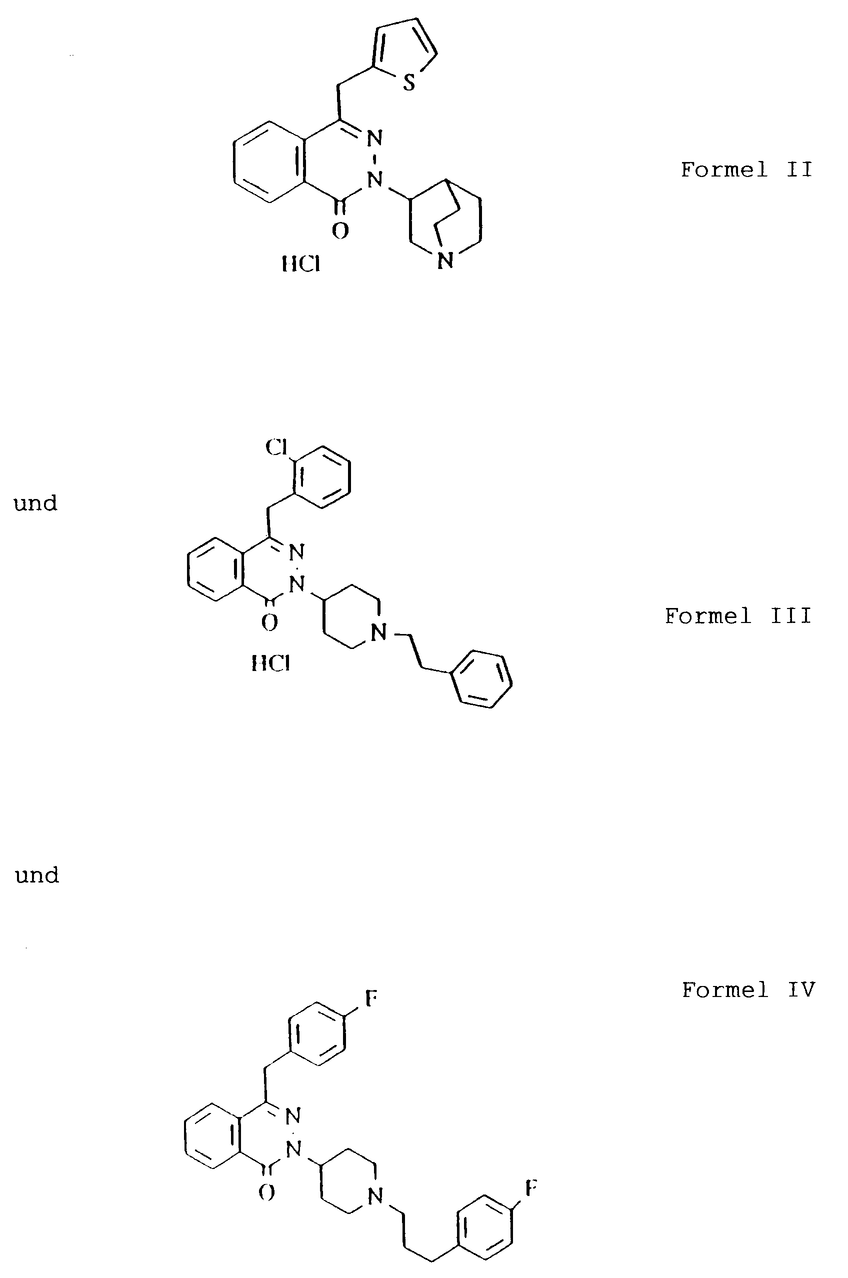 eine starke multi-drug-resistance durchbrechende Wirkung besitzen.

Bei der Therapie mit Zytostatika beobachtet man das Phänomen, daß der Tumor nach anfänglichen Behandlungserfolgen sich als therapieresistent erweist. Auch die Behandlung mit einem anderen Zystostatikum verspricht keinen Erfolg.

Dieses Phänomen bezeichnet man als multi-drug-resistance. (MDR) (Vendrik, Bergers, de Jong, Steerenberg Resistance to cytostatic drugs at the cellular level, Cancer Chemother, Pharmacol. (1992) 29, 413 - 429)

Die erfindungsgemäßen Verbindungen eignen sich auch zur Herstellung von Arzneimitteln gegen dieses Phänomen.

Die Eignung der Verbindungen zur Herstellung von Arzneimitteln, die die MDR durchbrechen, wurde mit der im folgenden beschriebenen Versuchsanordnung bestimmt.

Die akute Myeloid-Leukämie Brown Norway (BNML) ähnelt der akuten myelozytischen Leukämie beim Menschen hinsichtlich ihrer Wachstumgseigenschaften und ihrer Chemotherapie-Reaktionen (für eine neuere Übersicht zur BNML s. Martens et al., Leukemia 4, 241 - 257, 1990).
Um ein klinisch relevantes Arzneimittel-resistentes Leukämiemodell zu entwickeln, wurde eine von der BNML übernommene Zell-Linie mit der Bezeichnung LT12 ausgewählt. Diese BNML-Linie besitzt den Vorzug, daß sie in vivo und in vitro wachsen kann.

Durch Einführung (in vitro) des menschlichen mdr1-Gens in die Genom-DNA mittels Übertragung wurden die LT12-Zellen stoffresistent gemacht. Für diese Übertragung wurde, unter Kontrolle durch einen frühen Beschleuniger für einen CMV-Promotor, gefolgt von HBV Polyadenylations-Signalen, der Expressionsvector pFRCMV mdr1.6 verwendet, der eine ganze cDNA des menschlichen mdr1-Gens enthält. Die transfizierten DNA-Abschnitte wurden mit Mitoxantron selektiert und mit 200 nM Mitoxantron unter Stoffselektion gehalten.
Dieses Verfahren ergab eine stabile stoffresistente LT12-Zell-Linie, die als LT12/mdr bezeichnet wird.

### Endpunkt-Flowzytometrie

Mit Hilfe der Flowzytomertrie lassen sich Zellen aufgrund der waagerechten (Größenparameter) und senkrechten (Strukturparameter) Lichtstreuung identifizieren. Für jede Zelle werden drei Wirkungen bei Erregung durch 0.6 W eines
488-nm Laserstrahls gemessen: waagerechte Lichtstreuung, senkrechte Lichtstreuung (durch einen 488-nm Bandfilter) und die Daunorubicin-Fluoreszenz (durch einen 550-nm Langbandfilter).

Werden diese Steuerungswerte mit den Fluoreszenzwerten kombiniert, läßt sich der Daunorubicin-Gehalt der Zellenpopulation untersuchen.
Bei t=0 wurden Daunorubicin (Endkonzentration 2 µM) und eine Versuchsverbindung (Endkonzentration 1.0 µM, 3.0 µM oder 10 µM) der Zellensuspension zugesetzt (2x10⁵ Zellen/ml in RPMI-1640-Medium ohne Phenolrot). Die Zellen wurden 90 Minuten lang bei 37°C inkubiert. Mit Hilfe eines Flowzytometers wurde die intrazelluläre Daunorubicin-Konzentration durch Messung ihrer hellen Fluoreszenz bei Erregung mit 488-nm Laserstrahlung bestimmt.
RPMI-1640 Medium ohne Phenolrot diente als negative Kontrolle. In jedem Versuch wurde Cyclosporin A (0.3, 1 und 3 µM) als wirksamer Stoff verwendet.

Tote Zellen wurden mittels Gegenfärbung mit dem nichtvitalen Farbstoff Hoechst 33258 identifiziert (Erregung mit 0.2 W eines UV Laserstrahls durch einen 405 nm langen Durchgangsfilter). Diese toten Zellen und der Detritus wurden bei der Analyse immer ausgeschlossen.

### On-line Flowzytometrie

Die On-line Flowzytometrie ist eine Abwandlung der normalen
Flowzytometrie-Methode, die die ununterbrochene Messung einer Probe über einen Zeitraum von mehreren Stunden gestattet. Zu vorherbestimmten Zeitpunkten speichert der Versuchs-Microcomputer die Daten von jeweils 2000 Zellen in einer sogenannten List-Mode-Datei. Da alle relevanten Parameter (z.B. Größe, Struktur, Stoffakkumulation etc.) jeder Zelle gespeichert werden, lassen sich anschließend umfassende Datenanalysen und erneute Versuchsdurchläufe vornehmen.
Die Zellensuspension wird in einem Reaktionsgefäß mit einem thermostatischen Wassermantel, das mit der Flußküvette des Flowzytometers verbunden ist, bei 37°C gehalten. Das Medium, das die Zellen enthält, wird mittels Luftdruck durch die Flußküvette gepreßt. Ein weiterer Einlaß des Reaktionsglases gestattet die Zugabe von Stoffen, während die Zellen beobachtet werden. Die Methode der On-line-Flowzytometrie ist daher für die Messung (schneller) kinetischer Veränderungen intrazellulärer Stoffkonzentrationen besonders geeignet. Bei diesem Versuchstyp wurde die Messung ohne Daunorubicin in der Zellensuspension begonnen (2x10⁵ Zellen/ml in RPMI-1640-Medium ohne Phenolrot). Bei t=0 wurde den Zellen Daunorubicin (Endkonzentration 2 µM) beigegeben. Die Nettoaufnahme des Daunorubicin durch die Zellen wurde anschließend über einen Zeitraum von 60 Minuten gemessen, bis ein gleichmäßges Niveau der Stoffakkumulation erreicht war.

Im diesem Augenblick wurde der Zellensuspension die Testverbindung zugegeben, und die Stoffakkumulation wurde nochmals 60 Minuten lang gemessen. Der Zellensuspension wurde als positive Kontrollsubstanz Cyclosporin A beigefügt.

Die Ergebnisse belegen die Eignung der erfindungsgemäßen Verbindung zu diesem Zweck. Die LT12/mdr-Zellen zeigen unbehandelt einen Wert von 23 % Daunorubicin-Fluoreszenz, nicht resistente Zellen zeigen 100 % Daunorubicin-Fluoreszenz.
Die Vergleichssubstanz Cyclo-A zeigt bei der Konzentration von 0,3 µMol 120 %, bei 1,0 µMol 110 % und bei 3 µMol 130 %
Daunorubicin-Fluoreszenz.

Flezelastin ergibt bei 0,1 µMol 60 %, bei 0,3 µMol 110 %, bei 0,5 µMol 130 %, bei 1,0 µMol 130 % und bei 3,0 µMol 140 % Daunorubicin-Fluoreszenz.

Azelastin zeigt bei 0,1 µMol 50 %, bei 0,3 µMol 70 %, bei 0,5 µMol 90 %, bei 1,0 µMol 110 % und bei 3,0 µMol 140 % Daunorubicin-Fluoreszenz.

In J. Clin. Biochem. Nutr. 6, 205 - 111, 1989 wird von Yamamoto u.a. eine Verhinderung der Adriamycin (ADM) - Resistenz von Leulämie-Zellen durch Azelastin beschrieben.

Die Darstellung der Verbindungen gemäß Formel I und II kann gemäß DE 21 64 058 erfolgen.
Die Verbindung gemäß Formel III wird analog zum Beispiel 2 in EP 174 464 dargestellt.
Die Synthese der Verbindung gemäß Formel IV kann gemäß EP 0 222 191 , Beispiel 36 erfolgen.

Die optischen Isomeren von Flezelastin wurden mit Hilfe der Methode der fraktionierten Kristallisation gewonnen.

## Patentansprüche

1. Verwendung der Verbindungen gemäß Formel I 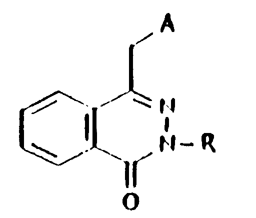
wobei A = Phenyl (unsubstituiert, ein- oder mehrfach halogensubstituiert),
H oder C₁-C₃-Alkyl, 2-Furyl oder 2-Thienyl bedeutet und
R = 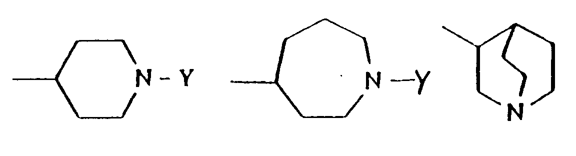 mit Y = C₁-C₆-Alkyl, wobei der Alkylrest mit einem (substituierten) Phenylring substituiert sein kann,
wenn R ein Azepinring bedeutet, dann ist Y = C₁-C₆-Alkyl, substituiert mit einem (substituierten) Phenylring.
sowie deren physiologisch unbedenkliche Säureadditionssalze
zur Herstellung eines Arzneimittels zur Durchbrechung der multi-drug-resistance.

2. Verwendung der Verbindung gemäß Formel II 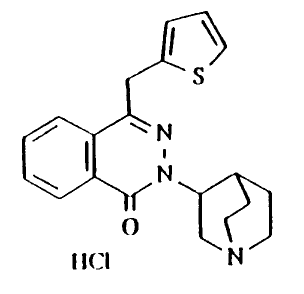 zur Herstellung eines Arzneimittels zur Durchbrechung der multi-drug-resistance.

3. Verwendung der Verbindung gemäß Formel III 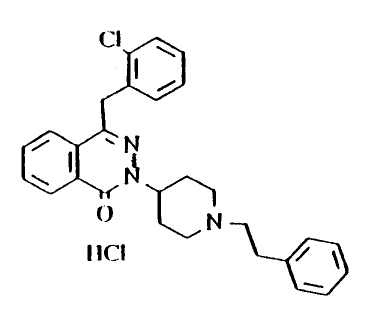 zur Herstellung eines Arzneimittels zur Durchbrechung der multi-drug-resistance.

4. Verwendung der Verbindung gemäß Formel IV 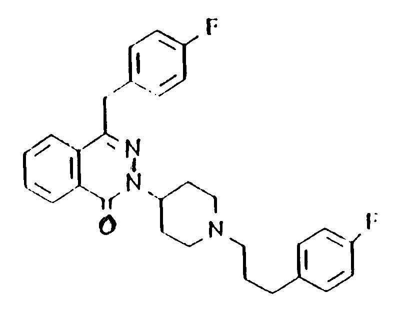 zur Herstellung eines Arzneimittels zur Durchbrechung der multi-drug-resistance.

5. Verwendung von Flezelastin sowie seinen optischen Isomeren zur Herstellung eines Arzneimittels zur Durchbrechung der multi-drug-resistance.

6. Verwendung des reinen (+)-Isomers von Flezelastin zur Herstellung eines Arzneimittels zur Durchbrechung des multi-drug-resistance.

7. Verwendung des reinen (-)-Isomers von Flezelastin zur Herstellung eines Arzneimittels zur Durchbrechung der multi-drug-resistance.

## Claims

1. Use of the compounds according to formula I 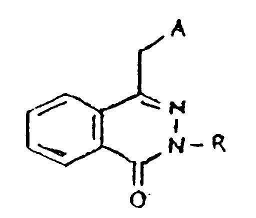
wherein A = signifies phenyl (unsubstituted, singly or multiply halogen-substituted),
H or C₁-C₃ alkyl, 2-furyl or 2-thienyl and
R = 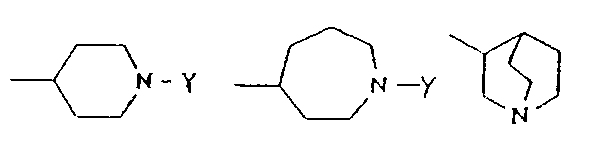 wherein Y is C₁-C₆ alkyl wherein the alkyl radical can be substituted by a (substituted) phenyl ring,
when R signifies an azepine ring, Y is C₁-C₆ alkyl substituted by a (substituted) phenyl ring,
and the physiologically acceptable acid addition salts thereof
for the preparation of a medicine for overcoming multi-drug resistance.

2. Use of the compound according to formula II 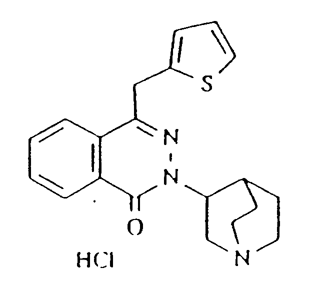 for the preparation of a medicine for overcoming multi-drug resistance.

3. Use of the compound according to formula III 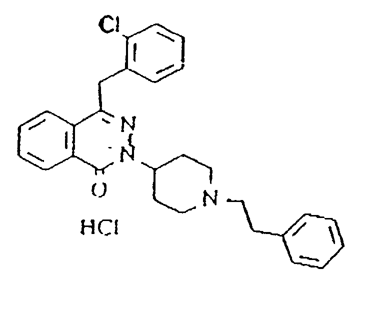 for the preparation of a medicine for overcoming multi-drug resistance.

4. Use of the compound according to formula IV 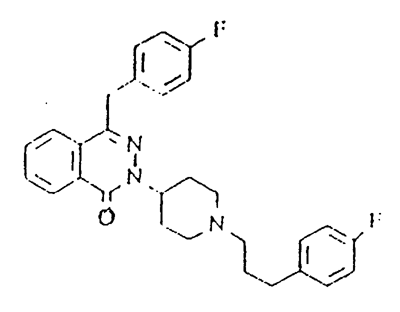 for the preparation of a medicine for overcoming multi-drug resistance.

5. Use of flezelastine and of its optical isomers for the preparation of a medicine for overcoming multi-drug resistance.

6. Use of the pure (+)-isomer of flezelastine for the preparation of a medicine for overcoming multi-drug resistance.

7. Use of the pure (-)-isomer of flezelastine for the preparation of a medicine for overcoming multi-drug resistance.

## Revendications

1. Utilisation des composés selon la formule I, 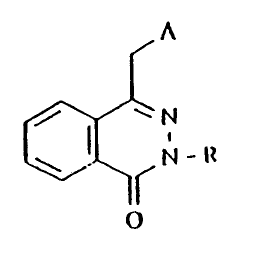 dans laquelle A = signifie un phényle (non substitué ou substitué une ou plusieurs fois par un halogène)
un H ou un alcoyle en C₁-C₆ un 2-thiényle ou 2-furyle.
R = 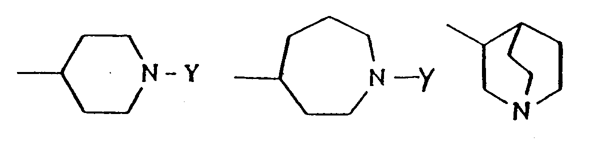
avec
Y = un alcoyle en C₁-C₆ dans lequel le radical alcoyle peut être substitué par un cycle phényle (substitué),
lorsque R signifie un cycle Azépine, alors
Y = un alcoyle en C₁-C₆ est substitué par un cycle phényle (substitué),
ainsi que leurs sels d'addition avec un acide physiologiquement inoffensif, pour la réalisation d'un médicament en vue d'interrompre la résistance multiple aux médicaments (multi-drug-resistance).

2. Utilisation du composé selon la formule II, 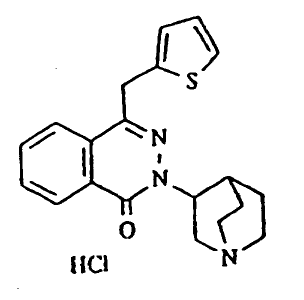 pour la réalisation d'un médicament en vue d'interrompre la résistance multiple aux médicaments.

3. Utilisation du composé selon la formule III, 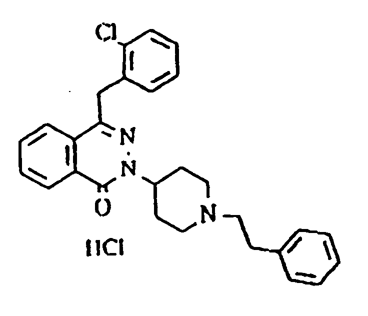 pour la réalisation d'un médicament en vue d'interrompre la résistance multiple aux médicaments.

4. Utilisation du composé selon la formule IV, 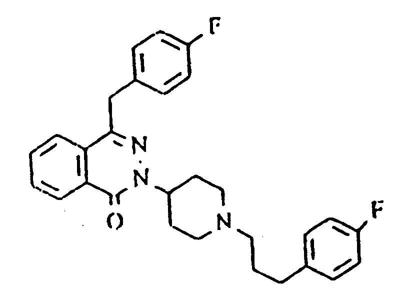 pour la réalisation d'un médicament en vue d'interrompre la résistance multiple aux médicaments.

5. Utilisation de la Flézélastine ainsi que de ses isomères optiques pour la réalisation d'un médicament en vue d'interrompre la résistance aux médicaments.

6. Utilisation de l'isomère pur (+)- de la Flézélastine pour la réalisation d'un médicament en vue d'interrompre la résistance multiple aux médicaments.

7. Utilisation de l'isomère pur (-)- de la Flézélastine pour la réalisation d'un médicament en vue d'interrompre la résistance multiple aux médicaments.
